# EUROPEAN PATENT SPECIFICATION

(11) **EP 2 253 323 B1**
(45) Date of publication and mention of the grant of the patent: **10.04.2019**
(21) Application number: 09714579.1
(22) Date of filing: 26.02.2009
(51) Int. Cl.: A61K 35/74, A61K 35/20, A61P 11/02, A61P 11/06, A61P 17/00, A61P 27/14, A61P 37/08

(54) **ANTI-ALLERGIC AGENT**
ANTIALLERGENER WIRKSTOFF
AGENT ANTIALLERGIQUE

(30) Priority: 29.02.2008 JP 2008049714
(43) Date of publication of application: 24.11.2010
(73) Proprietor: Meiji Co., Ltd., Chuo-ku Tokyo 104-8306 (JP)
(72) Inventor: KANO, Hiroshi, Odawara-shi Kanagawa 250-0862 (JP); IKEGAMI, Shuji, Odawara-shi Kanagawa 250-0862 (JP); FURUICHI, Keisuke, Odawara-shi Kanagawa 250-0862 (JP); ITOU, Hiroyuki, Odawara-shi Kanagawa 250-0862 (JP); ORII, Naoki, Odawara-shi Kanagawa 250-0862 (JP)
(74) Representative: Blodig, Wolfgang
(86) International application number: PCT/JP2009/000851
(87) International publication number: WO 2009/107380

(56) References cited:
- EP-A1- 1 738 761
- EP-A1- 1 852 498
- EP-A1- 1 974 720
- WO-A1-02/060276
- WO-A1-2005/072718
- WO-A1-2007/054989
- WO-A1-2008/079009
- WO-A2-2009/018447
- FR-A1- 2 889 057
- JP-A- 7 227 207
- JP-A- 10 304 871
- JP-T- 2005 529 960
- SARKAR S ET AL: "PROCESS FOR THE MANUFACTURE OF A NEW MODIFIED CULTURED MILK PRODUCT FOR INFANTS AND CHILDREN", MILCHWISSENSCHAFT, VV GMBH VOLKSWIRTSCHAFTLICHER VERLAG. MUNCHEN, DE, vol. 53, no. 11, 1 January 1998 (1998-01-01), pages 603-605, XP000799389, ISSN: 0026-3788
- OKADA Y ET AL: "Propionibacterium freudenreichii component 1.4-dihydroxy-2-naphthoic acid (DHNA) attenuates dextran sodium sulphate induced colitis by modulation of bacterial flora and lymphocyte homing", GUT, BRITISH MEDICAL ASSOCIATION, LONDON, UK, vol. 55, no. 5, 1 May 2006 (2006-05-01), pages 681-688, XP002666988, ISSN: 0017-5749, DOI: 10.1136/GUT.2005.070490 [retrieved on 2005-11-18]
- HIROSHI KANO ET AL.: 'Propion-san Kin Hakko Taisha Sanbutsu no Datsu Karyu Yokusei Sayo' NIPPON NOGEI KAGAKUKAI TAIKAI KOEN YOSHISHU vol. 2006, 05 March 2006, page 43, XP008138962
- HIROSHI KANO ET AL.: 'Atopy-sei Hifuen Model ni Okeru Propion-san Kin Hakkobutsu no Yokusei Sayo' NIPPON NOGEI KAGAKUKAI TAIKAI KOEN YOSHISHU vol. 2008, 05 March 2008, page 134, XP008138963

## Description

### Technical Field

The present invention relates to an antiallergic agent for use in improving dry skin conditions or skin roughness by oral administration or oral ingestion, wherein the fermentation product comprises a culture solution of a propionic acid bacterium.

### Background Art

In recent years, allergic symptoms such as rhinitis, conjunctivitis, bronchial asthma, and atopic dermatitis caused by foods, specific pollens such as Japanese cedar pollen, house dust, and chemical substances have become a social problem. Inparticular, along with attaining a higher airtightness of a house, house dust such as mites, dead bodies and feces thereof, molds, pollens, and pet hairs , which are caused by keeping pets indoors and the continuous use of an air conditioner, and chemical substances such as volatile organic compounds, which are caused by the use of volatile organic compounds such as formaldehyde and toluene during house construction, may exist indoors for a long period of time to cause the allergic symptoms. This has become a problem.

In general, the alleviation of the allergic symptoms greatly depends on medicaments such as antihistamines and steroids, which places a tremendous burden on a patient in terms of costs and adverse effects. Therefore, there has been demanded the alleviation of the allergic symptoms through the relatively simple ingestion of high-safety products derived from foods.

For example, it has been confirmed that a persimmon leaf extract is effective for a passive cutaneous anaphylaxis (PCA) reaction and an atopic dermatitis model (Patent Document 1). Further, it has been also confirmed that a microorganism has such effectiveness. However, the microorganism is genetically-modified *Escherichia coli* and needs to be sufficiently verified on its safety (Patent Document 2). In addition, there is a demand for searching foods, naturally-occurring microorganisms with high safety, and the like which are capable of alleviating the allergic symptoms.

By the way, a propionic acid bacterium (*Propionibacterium*) and lactic acid bacteria belonging to the genera *Lactococcus* and *Leuconostoc* have been conventionally used in the production of foods and beverages. It has also been reported that a culture solution of those bacteria contains 1,4-dihydroxy-2-naphthoic acid (DHNA) (Patent Document 3 and Non-patent Document 1).
[Patent Document 1] JP-A-2000-139406
[Patent Document 2] JP-A-2002-154976
[Patent Document 3] WO 03/016544 pamphlet

[Non Patent Document 4] K. Fruichi et al., Applied and Environmental Microbiology, May 2007, p. 3137-3143. EP 1974720 A1 discloses the use of a lipid of *Vitreoscilla filiformis* for the preparation of a composition to inhibit the proliferation of microbial flora to prevent and/or treat microbial infections and superinfections of the skin.

WO 2009018447 A2 discloses methods for characterization of bacterial skin microbiota to provide diagnostic, therapeutic, and preventive measures for alleviating skin conditions.

FR 2889057 A1 discloses a topical cosmetic or dermatological composition comprising a microorganism or its fraction or metabolite and a polyunsaturated fatty acid or its ester, salts or derivative.

### Disclosure of the Invention

### Problems to be Solved by the Invention

An obj ect of the present invention is to provide an antiallergic agent with high safety.

### Means for Solving the Problems

The inventors of the present invention have investigated substances capable of alleviating allergic symptoms and, as a result, have found that a fermentation product of a propionic acid bacterium has an action of alleviating atopic dermatitis and a passive cutaneous anaphylaxis (PCA) reaction caused by a mite-derived antigen, and that the fermentation product is useful as an antiallergic agent.

Accordingly, the present invention provides the subject matter of claims 1 to 6, in particular a fermentation product of a propionic acid bacterium for use in improving dry skin conditions or skin roughness by oral administration or oral ingestion, wherein the fermentation product comprises a culture solution of a propionic acid bacterium.

### Effects of the Invention

The present invention employs a fermentation product of a naturally-occurring microorganism conventionally used in the production of foods, and hence is useful as an antiallergic agent with high safety. Further, the use of the microorganism allows the mass production of the fermentation product as well.

### Brief Description of the Drawings

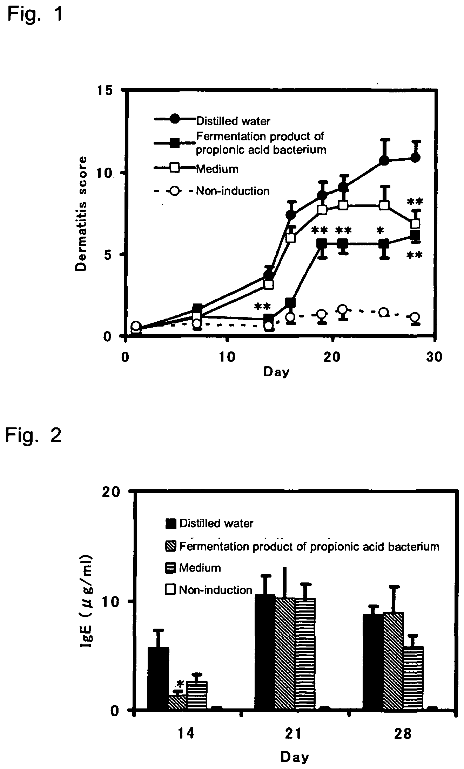

Fig. 1 shows transition of dermatitis symptom scores during a test period in a group that received distilled water [DISTILLED WATER], a group that received a fermentation product of a propionic acid bacterium [FERMENTATION PRODUCT of PROPIONIC ACID BACTERIUM], a group that received a medium (non-fermentation product) [MEDIUM], and a dermatitis non-induction group [NON-INDUCTION] (expressed as average±S.E. **P<0.01, *P<0.05, Dunnett's multiple comparison, a comparison with the group that received distilled water).
Fig. 2 shows total IgE concentrations in serum on days 14, day 21, and day 28 in a group that received distilled water [DISTILLED WATER], a group that received a fermentation product of a propionic acid bacterium [FERMENTATION PRODUCT of PROPIONIC ACID BACTERIUM], a group that received a medium (non-fermentation product) [MEDIUM], and a dermatitis non-induction group [NON-INDUCTION] (expressed asaverage±S.E. *P<0.05, Dunnett's multiple comparison, a comparison with the group that received distilled water).

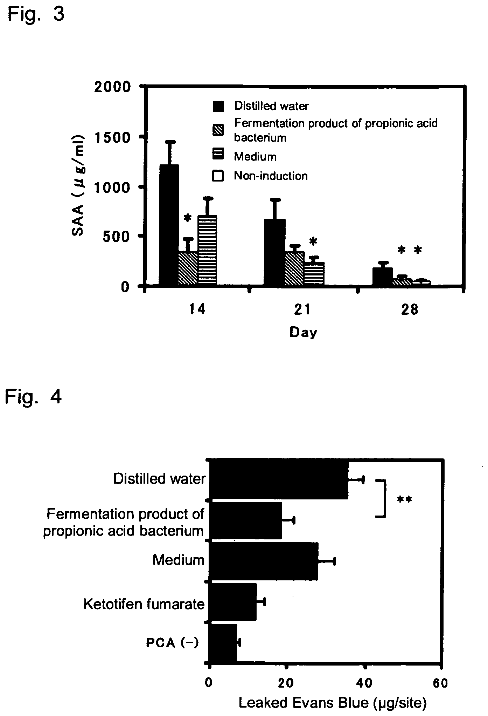

Fig. 3 shows SAA concentrations on days 14, day 21, and day 28 in a group that received distilled water [DISTILLED WATER], a group that received a fermentation product of a propionic acid bacterium [FERMENTATION PRODUCT of PROPIONIC ACID BACTERIUM], a group that received a medium (non-fermentation product) [MEDIUM], and a dermatitis non-induction group [NON-INDUCTION] (expressed as average±S.E. *P<0. 05, Dunnett'smultiple comparison, a comparison with the group that received distilled water).
Fig. 4 shows amounts of Evans Blue leakage in a PCA reaction induction site of a group that received distilled water [DISTILLED WATER], a PCA reaction induction site of a group that received a fermentation product of a propionic acid bacterium [FERMENTATION PRODUCT of PROPIONIC ACID BACTERIUM], a PCA reaction induction site of a group that received a medium [MEDIUM], a PCA reaction induction site of a group that received ketotifen fumarate [KETOTIFEN FUMARATE], and a PCA reaction non-induction site of each group [PCA (-)] (expressed as average±S.E. **P<0.01, Dunnett's multiple comparison, a comparison with the PCA reaction induction site of the group that received distilled water [DISTILLED WATER]).

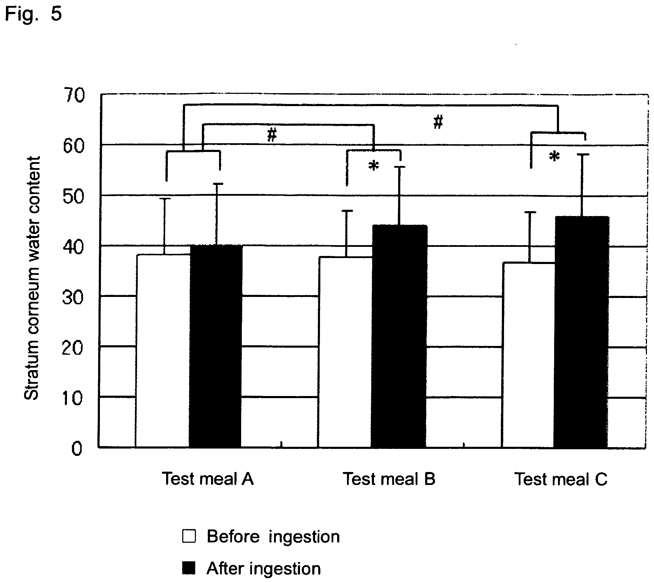

Fig. 5 shows stratum corneum water contents (AU) in the ingestion groups: Test meal A: an acidic beverage (placebo); Test meal B: an acidic beverage containing a whey fermentation product of a propionic acid bacterium (Active 1) ; and Test meal C: a yogurt beverage containing a whey fermentation product of a propionic acid bacterium (Active 2) (expressed as average±S.D. *P<0.05 (paired t-test), #P<0.05 (a multiple comparison test among three groups according to Tukey-Kramer's method)).

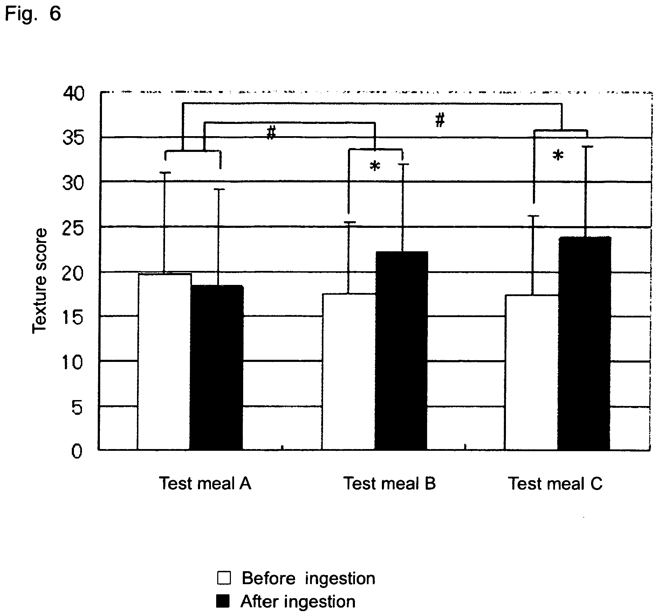

Fig. 6 shows texture scores in the ingestion groups : Test meal A: an acidic beverage (placebo); Test meal B: an acidic beverage containing a whey fermentation product of a propionic acid bacterium (Active 1); and Test meal C: a yogurt beverage containing a whey fermentation product of a propionic acid bacterium (Active 2) (expressed as average±S.D. *P<0.05 (paired t-test), #P<0.05 (a multiple comparison test among three groups according to Tukey-Kramer's method)).

### Best Modes for Carrying Out the Invention

Examples of the propionic acid bacterium used in the present invention include, but are not particularly limited to, bacteria belonging to the genera *Propionibacterium, Propionicimonas, Propioniferax, Propionimicrobium,* and *Propionivibrio.* The bacteria belonging to the genus *Propionibacterium* are preferred. Examples of the bacteria belonging to the genus *Propionibacterium* include propionic acid bacteria for cheese such as *P. freudenreichii* (or *Propionibacterium freudenreichii*)*, P. thoenii* (or *Propionibacterium thoenii*)*, P. acidipropionici* (or *Propionibacterium acidipropionici*)*,* and *P. jensenii* (or *Propionibacterium jensenii*), *P. avidum, P. acnes, P. lymphophilum, P. granulosam, P. arabinosum, P. cyclohexanicum, Propionibacterium innocuum, Propionibacterium intermediu, Propionibacterium pentosaceum, Propionibacterium peterssonii, Propionibacterium propionicum,* and *Propionibacterium zeae.* The propionic acid bacteria for cheese are preferred. Of these, *Propionibacterium freudenreichii* is more preferred and *Propionibacterium freudenreichii* ET-3 strain (the strain deposited in International Patent Organism Depositary of National Institute of Advanced Industrial Science and Technology (Central 6, 1-1-1 Higashi, Tsukuba, Ibaraki, Japan (postal code 305-8566) as an accession No. FERMBP-8115 on August 9, 2001; the identification reference is ET-3; and the strain was transferred to deposit based one Budapest Treaty on July 11, 2002) is particularly preferred.

A nutrient for a medium to be used in a culturing step which is a part of a production process for the fermentation product of a propionic acid bacterium to be used according to the present invention may be any product to be used in the culture of a propionic acid bacterium (for example, Patent Document 3 and Non-patent Document 1), and a dairy product, a yeast extract, a soybean extract, peptone such as trypticase, and a food containing large amounts of those nutrients may be used. Alternatively, an enzyme-treated product thereof may be used. Of these, a medium containing a proteolytic enzyme-treated dairy product is preferred. More preferably, the medium contains the proteolytic enzyme-treated dairy product as a main ingredient. The content of the proteolytic enzyme degradation product in a liquid medium is more preferably 0.1 to 40% by mass and even more preferably 1 to 20% by mass.

Examples of the dairy product to be used for the proteolytic enzyme degradation product include fresh milk, concentrated milk, whole milk powder, skim milk, skim milk powder, a milk protein (MPC (milk protein concentrate)), casein, whey, concentrated whey, a whey protein concentrate (WPC), a whey protein isolate (WPI), and whey powder, and they may be used alone or in combination of two or more kinds. Of these, skim milk, skim milk powder, concentrated whey, whey powder, and a milk protein are preferred. Commercially-available products thereof may be used. The use amount of the dairy product in a liquid medium is preferably 0.1 to 40% by mass and more preferably 1 to 20% by mass.

Further, as the proteolytic enzyme, a commercially-available product thereof may be used, and protease is preferred. Of these, neutral protease is preferred, and the proteolytic enzyme may include peptidase in addition to protease. The protease or peptidase to be used is not particularly limited. Examples of food-grade protease include an endoprotease, an exoprotease, an exopeptidase/endoprotease enzyme mixture, and a protease/peptidase enzyme mixture. Examples of the endoprotease include chymosin (EC 3.4.23.4, Maxiren, derived from modified yeast *Kluyveromyces lactis,* GIST-BROCADESN.V.), AlcalaseR (derived from *Bacillus licheniformis,* Novo Nordisk A/S), Esperase (derived from *B. lentus,* Novo Nordisk A/S), NeutraseR (derived from *B. subtilis,* Novo Nordisk A/S), Protamex (derived from bacteria, Novo Nordisk A/S), and PTN6.0S (trypsin derived from porcine pancreas, Novo Nordisk A/S) . Examples of the exopeptidase/endoprotease enzyme mixture include Flavorzyme (derived from *Aspergillus oryzae,* Novo Nordisk A/S). In addition, examples of the endoprotease include trypsin (CAS No. 9002-07-7, EC 3.4.21.4, derived from bovine pancreas, Product No. T8802, SIGMA), pepsin (CAS No. 9001-75-6, EC 3.4.4.1, derived from porcine stomach mucous membrane, SIGMA), chymotrypsin (Novo Nordisk A/S, Boehringer Ingelheim GmbH), Protease N "Amano" G (derived from *Bacillus subtilis;* Amano Enzyme Inc.), Bioplaze (derived from *Bacillus subtilis;* NAGASE & CO., LTD.), and Papain W-40 (Amano Enzyme Inc.). Examples of the exoprotease include pancreatic carboxypeptidase and aminopeptidase in small intestine brush border. Further, as the protease/peptidase enzyme mixture, for example, Protease A "Amano" G (derived from *Aspergillus oryzae;* Amano Enzyme Inc.) and Umamizyme G (peptidase and protease, derived from *Aspergillus oryzae;* Amano Enzyme Inc.) may be used. The origin of the enzyme is not limited to the above description. The enzyme may be derived from any of animals, plants, and microorganisms, and is suitably derived from *Aspergillus oryzae.* Those enzymes do not mean the limitation of trade names, origins, manufacturers, and the like. In an embodiment of the present invention, one enzyme may be used or two or more kinds of the enzymes may be used in combination. A suitable example thereof may include protease A "Amano" G (derived from *Aspergillus oryzae;* Amano Enzyme Inc.), but is not limited to this example. Further, when the enzymes as mentioned above are used in combination, each enzyme reaction may be carried out simultaneously or separately. The use amount of the enzyme may be an amount capable of degrading proteins in a dairy product, and is preferably 0.01 to 10 parts by mass and more preferably 0.1 to 10 parts by mass with respect to 100 parts by mass of the dairy product.

A production method for the proteolytic enzyme-treated dairy product involves: adding a proteolytic enzyme to a dairy product; and subjecting the resultant to heat treatment to degrade proteins in the dairy product. The pH, enzyme degradation time, and enzyme reaction temperature at the time of enzyme degradation onset are not particularly limited as long as a product to be used according to the present invention is obtained.

The heating temperature may be any temperature at which a proteolytic enzyme is activated, and is preferably 20 to 60°C, more preferably 40 to 60°C, and still more preferably 40 to 50°C.

The heating time is not particularly limited, and is preferably 1 to 10 hours and more preferably 3 to 8 hours.

The pH (25°C) is not particularly limited, and is preferably 2 to 8 and more preferably 5 to 8. Examples of buffers for adjusting pH include salts of organic acids such as carbonic acid, acetic acid, citric acid, fumaric acid, malic acid, lactic acid, gluconic acid, and tartaric acid, salts of inorganic acids such as phosphoric acid, hydrochloric acid, and sulfuric acid, hydroxides such as sodium hydroxide, ammonia, and aqueous ammonia, and they may be used alone or in combination of two or more kinds.

Hereinafter, there is described an example of a production method for the proteolytic enzyme degradation product of a dairy product.

Skim milk powder and a milk protein are dissolved or suspended in water to prepare a solution so as to achieve the respective concentrations of 3 to 6% (w/w) and 3 to 6% (w/w) . To the solution, 0.25 to 5 parts by mass of protease are added with respect to 100 parts by mass of the dairy product to prepare a mixed solution. The mixed solution is subjected to the degradation of proteins in the dairy product at 40 to 50°C for 5 to 7 hours at pH 6 to 8, whereby a protease-treated dairy product (proteolytic enzyme degradation product) may be obtained.

Further, whey powder (10 w/w%) and Protease Amano A "Amano" G (0.07 w/w%, manufactured by Amano Enzyme Inc.) are dissolved in water, subjected to enzyme degradation at 47°C (pH 6.6) for 2 hours, and then heated at 85°C for 10 minutes to inactivate the enzyme, whereby a proteolytic enzyme degradation product of a dairy product may be also obtained, but the production method is not limited to this example.

To a medium to be used, in addition to the proteolytic enzyme-treated dairy product, any ingredient such as trypticase, phytone, a yeast extract, and a carbohydrate may be appropriately added. Of these, at least the yeast extract is preferably added, and the use amount of the yeast extract is preferably 0.1 to 10 parts by mass and more preferably 0.5 to 10 parts by mass with respect to 100 parts by mass of the dairy product.

As the carbohydrate, there may be also used lactose or a lactase-treated product thereof, glucose, fructose, sucrose, or sugar wastewater, and of these, lactose is preferred. A mineral such as a whey mineral may be contained.

A commercially-available product may be used as any ingredient above, or a food containing large amounts of them may be used.

An example of the medium to be used includes a product obtained by the following: in an appropriate amount of ion-exchange water, dissolving 3% (w/w) skim milk powder (Meiji Dairies Corporation), 3.4% (w/w) Milk Protein Concentrate (MPC: Murray Goulburn), and 0.25% (w/w) protease (Protease A "Amano" G, manufactured by Amano Enzyme Inc.) with respect to the amount of the medium; degrading proteins contained in the skim milk powder and the MPC with the enzyme at 47°C for 6 hours; and after the degradation, adding a 0.5% (w/w) brewer's yeast extract (P2G, manufactured by ASAHI FOOD & HEALTHCARE CO., LTD.) and adjusting pH to 6.8 to 6.9, followed by sterilization at 121°C for 15 minutes. The pH at the time of enzyme degradation is adjusted to 6.6 to 7.0 with a solution of potassium carbonate.

Another example of the medium to be used includes a product obtained by the following: dissolving whey powder (10 w/w%) and Protease Amano A "Amano" G (0.07 w/w%, manufactured by Amano Enzyme Inc.) in water; degrading the resultant with the enzyme at 47°C (pH 6.6) for 2 hours; thereafter heating at 85°C for 10 minutes to thereby inactivate the enzyme; and then adding a brewer's yeast extract (0.10 w/w%, manufactured by ASAHI FOOD & HEALTHCARE CO., LTD.) and ammonium sulfate (0.27 w/w%) and adjusting pH to 6.7, followed by sterilization at 121°C for 7 minutes, but is not limited to this example.

The fermentation product of a propionic acid bacterium to be used according to the present invention obtained by using, as a nutrient, a product derived from a milk protein, such as fresh milk, concentrated milk, whole milk powder, skim milk, skim milk powder, a milk protein (milk protein concentrate (MPC)), casein, concentrated whey, or whey powder is hereinafter referred to as a milk protein fermentation product of a propionic acid bacterium. In addition, the fermentation product of a propionic acid bacterium obtained by using, as a nutrient, a product derived from whey, such as whey, WPC, or WPI is hereinafter referred to as a whey fermentation product of a propionic acid bacterium.

As a culture method, various known aerobic or anaerobic culture methods may be employed, and the aerobic or anaerobic culture methods using liquid media are preferred from the viewpoint of mass production.

Further, for the purpose of enhancing the production efficiency of the fermentation product of a propionic acid bacterium, it is preferred that the number of cells of the propionic acid bacterium be preliminarily increased in a preculture under an anaerobic condition, followed by a main culture under an anaerobic or aerobic condition.

In view of the propionic acid bacterium being anaerobic, there are employed more preferably the anaerobic culture method and even more preferably the anaerobic culture method followed by the aerobic culture method.

The culture temperature during the culture in the anaerobic culture method is preferably 20 to 40°C and more preferably 30 to 40°C. Further, the pH of the medium is preferably neutral to slightly acidic, and specifically, is adjusted to preferably 5.0 to 8.0, more preferably 6.0 to 7.0, and even more preferably 6.2 to 6.8. The culture period is preferably 1 to 12 days and more preferably 5 to 12 days.

The culture temperature during the culture in the aerobic culture method is preferably 20 to 40°C and more preferably 30 to 40°C. Further, the pH of the medium is preferably neutral to slightly acidic, and specifically, is adjusted to preferably 5.0 to 8.0, more preferably 6.0 to 7.0, and even more preferably 6.2 to 6.8. The culture period is preferably 1 to 10 days and more preferably 3 to 7 days.

The fermentation product of a propionic acid bacterium as an active ingredient to be used according to the present invention is obtained by culturing the propionic acid bacterium using the above-mentioned medium by the above-mentioned culture method and contains both the propionic acid bacterium and the culture.

The obtained fermentation product of a propionic acidbacterium may be used as is, and may be appropriately subjected to concentration, dilution, drying, or the like.

Here, the addition of an antioxidant may prevent the produced DHNA from being converted into 2-amino-3-carboxy-1,4-naphthoquinone (ACNQ).

Here, as the antioxidant, there may be used ascorbic acid or a salt thereof, an ascorbic acid ester, erythorbic acid, or the like, and ascorbic acid or a salt thereof is particularly preferred. Examples of the salt include a salt of alkali metal such as sodium and potassium; a salt of alkali earth metal such as calcium and magnesium; and a salt of ammonia.

The content of the antioxidant in the fermentation product of a propionic acid bacterium is preferably 0.001 to 10% by mass, more preferably 0.01 to 5% by mass, and particularly preferably 0.04 to 3% by mass.

The propionic acid bacterium in the fermentation product may be any of a viable bacterium, a killed bacterium, or a ground product thereof.

In this case, the content of the propionic acid bacterium in 1 mL of the fermentation product is preferably 1 to 9×10¹⁰ cfu and morepreferably2 to 6×10¹⁰ cfu. Further, the content of the propionic acid bacterium in 1 mL of the fermentation product in terms of dry mass is preferably 50 to 500 mg and more preferably 100 to 300 mg.

Hereinafter, there is described an example of a production method for the fermentation product of a propionic acid bacterium to be used according to the present invention.

A precultured culture solution containing a propionic acid bacterium was inoculated into a culture solution (1 to 3 L) followed by a main culture.

During the culture at 30 to 40°C, while the culture solution being sparged with nitrogen gas at 0.1 to 1 L/min, an anaerobic culture is performed at a stirring speed of 50 to 200 rpm for 140 to 160 hours. In this case, after 1 to 2 days culture following the inoculation of the propionic acid bacterium, a carbohydrate may be sequentially added as any medium ingredient at 0.5 to 20 mL/h.

In addition, the anaerobic culture is preferably followed by the aerobic culture. In this case, the aeration is performed in the same manner as in the above by using oxygen gas instead of nitrogen gas, and the aerobic culture is performed for 110 to 130 hours.

In this way, the culture of the propionic acid bacterium provides a culture solution of the propionic acid bacterium. Further addition of an antioxidant into the culture solution allows the use of the culture solution as the fermentation product of a propionic acid bacterium.

Further, the fermentation product of a propionic acidbacterium as an active ingredient to be used according to the present invention may be also produced according to the known production methods described in WO 03/16544 pamphlet and the like. As used herein, the " fermentation product of a propionic acid bacterium" encompasses a culture itself obtained through fermentation by the propionic acid bacterium, a product obtained by adding an antioxidant to the culture, and a treated product thereof, for example, a culture filtrate or a culture supernatant obtained through filtration or decolonization of the culture, a concentrate obtained through concentration of the culture filtrate or the like with an evaporator or the like, a paste, a dilution, or a (freeze-)dried product.

Still further, the fermentation product of a propionic acid bacterium obtained as described above may be purified with, for example, adsorption chromatography of activated charcoal, an ion-exchange resin, or the like for the purpose of removing impurities in the fermentation product of a propionic acid bacterium or concentrating an active ingredient. Further, the fermentation product of a propionic acid bacterium may be diluted with a solvent to prepare a soluble fraction or an insoluble fraction. As the solvent, water and a generally-used solvent, for example, alcohols, hydrocarbons, organic acids, organic bases, inorganic acids, inorganic bases, and supercritical fluids may be used alone or in combination of multiple kinds.

The fermentation product of a propionic acid bacterium disclosed herein may alleviate allergic symptoms or the like caused by mites or chemical substances as described in Test Example 1 below, and hence may be used as an antiallergic agent for suppressing rhinitis, conjunctivitis, bronchial asthma, or atopic dermatitis. Atopic dermatitis is an immune-mediated inflammation of the skin, and specifically, superficial skin inflammation involving chronic itchiness. The atopic dermatitis includes both IgE-mediated (extrinsic) and non-IgE-mediated (intrinsic) types. Examples of an environmental factor of the atopic dermatitis include foods (for example, milk, eggs, wheat, peanuts, and fish), airborne allergens (for example, mites, molds, and dander). Further, it is also suggested that a hereditary factor involves in the development of atopic dermatitis (Fukushima Masanori, general editor, The Merck Manual, 18th ed., Japanese edition, published by Nikkei Business Publications, Inc., pp. 999 (2006)).

Further, as described in Test Example 3 below, the fermentation product of a propionic acid bacterium to be used according to the present invention increases a stratum corneum water content and a skin texture score, and hence is also useful for an agent for improving dry skin conditions, that is, an agent for preventing and/or improving skin roughness. In addition, a method for improving skin conditions through moisture retention on dry skin is as one method for treating atopic dermatitis, which indicates that the agent for preventing and/or improving skin roughness is useful for the treatment of atopic dermatitis. Therefore, the agent may be also used as an antiallergic agent. As used herein, the "skin roughness" refers to a state in which the stratum corneum water content decreases, resulting in a dry skin surface and a rough skin texture.

Further, the fermentation product of a propionic acid bacterium disclosed herein may be also used as various formulations, and may be also used to produce an antiallergic agent, an agent for preventing and/or improving skin roughness, and the like.

The antiallergic agent and the like may be utilized in any form of foods and beverages or pharmaceutical agents . For example, the antiallergic agent may be utilized as follows : the antiallergic agent may be directly administered as a pharmaceutical agent; or directly orally ingested as a food for special dietary use such as a food for specified health use, a food with nutrient function claims, or a supplement; or added to various foods (milk, fermented milk, yogurt, cheese, bread, a biscuit, a cracker, a pizza crust, modified milk powder, a fluid diet, a invalid diet, a nutritious food, a frozen food, a processed food, other foods on market, or the like, followed by the ingestion. Further, the antiallergic agent may be ingested in other forms such as tubal nutrition and enteral nutrition.

In the food containing the fermentation product of a propionic acid bacterium to be used according to the present invention, there may be used, as a main component, water, a protein, a carbohydrate, a lipid, vitamins, minerals, an organic acid, an organic base, fruit juice, a flavor, or the like.

Examples of the protein include: animal or plant proteins such as whole milk powder, skimmilkpowder, partially-skimmed milk powder, casein, whey powder, a whey protein, a whey protein concentrate, a whey protein-isolated product, α-casein, β-casein, κ-casein, β-lactoglobulin, α-lactoalbumin, lactoferrin, a soy protein, an egg protein, and a meat protein, and degraded products thereof; various milk-derived components such as butter, whey mineral, cream, whey, a non-proteinous nitrogen, sialic acid, a phospholipid, and lactose. The protein may contain a peptide or an amino acid such as a casein phophopeptide, arginine, and lysine.

Examples of the carbohydrates include sugars, processed starches (such as dextrin, a soluble starch, a British starch, an oxidized starch, a starch ester, and a starch ether), and dietary fibers. Examples of the lipid include: animal fat and oil such as lard, a fish oil, a separated oil thereof, a hydrogenated oil thereof, and an ester exchange oil thereof; plant fat and oil such as palm oil, safflower oil, corn oil, rape oil, coconut oil, a separated oil thereof, a hydrogenated oil thereof, and an ester exchange oil thereof.

Examples of the vitamins include vitamin A, carotenes, vitamin Bs, vitamin C, vitamin Ds, vitamin E, vitamin Ks, vitamin P, vitamin Q, niacin, nicotinic acid, pantothenic acid, biotin, inositol, choline, and folic acid. Examples of the minerals include calcium, potassium, magnesium, sodium, copper, iron, manganese, zinc, and selenium. Examples of the organic acid include malic acid, citric acid, lactic acid, tartaric acid, and erythorbic acid. These components may be used in combination of two or more kinds. In addition, a synthetic product and/or a food containing those compounds in a large amount may be used. The form of the food may be a solid or a liquid, and may be a gel.

Further, the fermentation product of a propionic acidbacterium to be used according to the present invention may be administered in various forms when being used as a pharmaceutical agent. Examples of the administration form include oral administration by using tablets, capsules, granules, powders, syrups, and the like. The fermentation product may be processed into formulations such as injections and solutions and then administered in other administration forms such as tubal administration and enteral administration. These various formulations may be formulated, according to a conventional method, by using a base compound and generally-used known adjuvants such as excipients, binders, disintegrants, lubricants, flavoring agents, solubilizing agents, suspending agents, and coating agents. In addition, an appropriate amount of vitamins, minerals, organic acids, sugars, amino acids, peptides, or the like may be added.

When the fermentation product of a propionic acid bacterium is used, the daily ingestion amount of the fermentation product of a propionic acid bacterium in terms of dry mass is preferably 10 to 500 mg, and more preferably 50 to 200 mg, per kg of the human body weight. Further, the daily ingestion amount in terms of the number of cells is preferably 10⁸ to 10¹² cfu, and more preferably 10⁹ to 10¹¹ cfu, per kg of the human body weight. Further, there may be used in combination with conventionally-known pharmaceutical agents and foods having an antiallergic action and an action of preventing and/or improving skin roughness.

### Examples

Production Example 1: Production method for fermentation product of propionic acid bacterium

### 1. Material

### <Strain>

### Propionibacterium freudenreichii ET-3 strain (FERM BP-8115 strain)

### <Preculture medium>

10% (w/w) whey powder (Meiji Dairies Corporation) and 0.07% (w/w) protease (Protease A "Amano" G: derived from *Aspergillus oryzae*: Amano Enzyme Inc.) with respect to the amount of the medium were dissolved in an appropriate amount of ion-exchange water, and proteins contained in the whey powder were degraded with the enzyme at 47°C for 3 hours. After the degradation, 0.1% (w/w) yeast extract (P2G: ASAHI FOOD & HEALTHCARE CO., LTD.) was added, and the pH was adjusted to 6.8 to 6.9, followed by sterilization at 121°C for 15 minutes. The pH at the time of enzyme degradation was adjusted to 6.6 to 7.0 with a solution of potassium carbonate.

### <Main culture medium>

3% (w/w) skim milk powder (Meiji Dairies Corporation), 3.4% (w/w) Milk Protein Concentrate (MPC: Murray Goulburn), and 0.25% protease (Protease A "Amano" G) with respect to the amount of the medium were dissolved in an appropriate amount of ion-exchange water, and proteins contained in the skim milk powder and the MPC were degraded with the enzyme at 47°C for 6 hours. After the degradation, 0.5% yeast extract (P2G) was added, and the pH was adjusted to 6.8 to 6.9, followed by sterilization at 121°C for 15 minutes. The pH at the time of enzyme degradation was adjusted to 6.6 to 7.0 with a solution of potassium carbonate.

### 2. Production method

### <Preculture>

To 100 mL of a preculture medium, 1 mL of frozen ET-3 strain (stored at -80°C) was inoculated. A static culture was performed at 37°C for 48 hours under an anaerobic condition, and the culture solution was used as a starter in a main culture.

### <Main culture>

To 2,000 mL of a main culture medium, 20 mL of the preculture solution were inoculated to start the main culture. The culture temperature was set to be 33°C, and the pH was adjusted to 6.5 with an aqueous solution of potassium carbonate. The aeration was performed by sparging nitrogen at 0.4 L/min, and the stirring speed was set to be 150 rpm. From 72 hours after the start of the culture, a 1.5 M solution of lactose (preliminarily sterilized at 121°C for 15 minutes) was fed at 0.90 mL/h for 192 hours . After the completion of the feeding, the aeration was changed from nitrogen to oxygen to perform an aerobic culture, and the aerobic culture was continued for 120 hours to complete the culture, whereby a culture solution containing a propionic acid bacterium was obtained.

After the completion of the culture, for the purpose of preventing DHNA produced by the propionic acid bacterium from being converted into ACNQ, 5 g of sodium ascorbate were added per 1,000 mL of the culture solution so as to allow ascorbic acid to coexist in the fermentation product, to thereby obtain a fermentation product of a propionic acid bacterium.

The production method for the fermentation product was carried out according to the method of Furuichi et al. (Furuichi et al., Enhancement of 1,4-dihydroxy-2-naphthoic acid production by Propionibacterium freudenreichii ET-3 fed-batch culture. Appl Environ Microbiol. 2007; 73(10):3137-43.).

In 0.5 mL of the fermentation product of a propionic acid bacterium after the above operation, 2.43×10¹⁰ cfu of the propionic acid bacterium were contained.

Through the following HPLC analysis after the above operation, 1, 4-dihydroxy-2-naphthoic acid (DHNA) was confirmed as a substance contained in the culture solution, the content of which was 0.34 mM (68 mg/L) per 1 L of the culture solution. In contrast, almost no 2-amino-3-carboxy-1,4-naphthoquinone (ACNQ) was observed.

### <HPLC analysis>

Column: Capcell pak C18 SG120, particle size: 5 µm, internal diameter: 4.6 mm, length: 250 mm (Shiseido Co., Ltd.)
Eluent: acetonitrile:methanol:water:acetic acid=250:100:900:0.6 (adjusted to pH 5.6 with 5% aqueous ammonia)
Flow rate: 1 mL/min
Injection volume: 10 µL
Detector: UV 270 nm

### <HPLC sample preparation method>

2.5 mL each of acetone and ethyl acetate were added to and mixed with 1 mL of the culture solution, and then the mixture was centrifuged at 2,000×g for 5 minutes . The supernatant was filtrated through a membrane filter.

### Test Example 1: Suppression test of atopic dermatitis (animal experimental model of atopic dermatitis)

### (1) Outline

Female 4-week-oldNC/Nga mice (Japan SLC, Inc.) were purchased and divided into the following four groups: dermatitis induction groups (a group that received distilled water (n=7) [DISTILLED WATER], a group that received a liquid medium (hereinafter also referred to as medium) (n=7) [MEDIUM] as a comparative example, and a group that received a fermentation product of a propionic acid bacterium (liquid) (n=7) [FERMENTATION PRODUCT OF PROPIONIC ACID BACTERIUM]) ; and a dermatitis non-induction group (n=7) [NON-INDUCTION]. After the grouping, various samples were orally administered daily (0.5 mL/mouse per day) during a test period (from day -7 to day 27).

In the various samples, the fermentation product of a propionic acid bacterium obtained in Production Example 1 above (containing 68 mg/L of DHNA and 4. 86×10¹⁰ cfu/ml of the propionic acid bacterium) was used, and the sterilized main culture medium non-inoculated with a propionic acid bacterium produced in Production Example 1 above was used as the liquid medium.

From 7 days after the start of the oral administration (day 0), dermatitis was induced by a method described later for the dermatitis induction groups, and the transition of the symptom was scored. Further, the serum was collected on 14, 21, and 28 days after the start of the dermatitis induction, the serum amyloid A (SAA) and IgE concentrations were measured by an ELISA method. The SAA concentration was measured with an ELISA kit manufactured by Bio Source International, Inc. The measurement was performed according to the accompanying instruction manual. The IgE concentration was measured with an antibody manufactured by BD Biosciences Pharmingen by partially modifying a protocol recommended by BD Biosciences Pharmingen.

### (2) Preparation of mite fracture solution

The whole body of *Dermatophagoides pteronyssinus* (Mite-Dp, manufactured by LSL Co., Ltd.) was degreased with anhydrous ether and then supplemented with distilled water, followed by ultrasonic fracture. Subsequently, the water-soluble fraction was centrifuged and lyophilized. After that, the resultant was adjusted with distilled water so as to achieve a protein concentration of 4.5 mg/ml (in terms of BSA, measured with DC protein assay manufactured by Bio-Rad Laboratories, Inc.) before use.

### (3) Induction of dermatitis

A method of Unno et al. was modified (Unno Tetsushi et al. , Allergy 50: 1152-1162, 2001) . Hairs at dermatitis induction sites (cephalic region, auricular region, and cervical region) were shaved with clippers, and then a 4% aqueous solution of SDS was applied onto the dermatitis induction sites of all the mice 20 times with a brush (the application amount corresponds to about 60 µL) . After drying of the aqueous solution of SDS, the mite fracture solution and distilled water were applied to the dermatitis induction groups and the dermatitis non-induction group, respectively, 20 times in the same manner as in the above. The above operation was repeated one set per day daily to initiate dermatitis.

### (4) Measurement of IgE

To a 96-well plate, an anti-IgE antibody (manufactured by BD Biosciences Pharmingen) as a primary antibody was added at 2 µg/mL and the plate was left to stand still at 37°C for 1 hour. After washing with 0.05% Tween20/PBS (PBS-Tween), 1% BSA/PBS was added and the plate was left to stand still at room temperature for 30 minutes. After washing with PBS-Tween, a serum sample and IgE (manufactured by BD Biosciences Pharmingen) for preparing a standard curve were added and the plate was left to stand still at room temperature for 30 minutes. After washing with PBS-Tween, a biotin-anti-IgE antibody as a secondary antibody (manufactured by BD Biosciences Pharmingen) was added in a concentration of 0.5 µg/mL and the plate was left to stand still at room temperature for 1 hour. AfterwashingwithPBS-Tween, streptavidin-HRP (manufactured by BD Biosciences Pharmingen) was added and the plate was left to stand still at room temperature for 30 minutes . After washing with PBS-Tween, TMB+Substrate Chromogen (manufactured by DAKO) was added to start a color development reaction. After the color development reaction, 1 N sulfuric acid was added, and an absorbance at 450 nm was measured with a microplate reader to calculate an IgE concentration in the serum.

### (5) Results

Fig. 1 shows transition of dermatitis symptom scores. The scoring was performed by evaluating the following five items: 1) dry skin and crust formation; 2) flare and bleeding; 3) tissue shedding and excoriation; 4) edema; and 5) pruritic action in the following four grades: 0 (none); 1 (slight); 2 (moderate); and 3 (severe), and calculating the total of each score in each mouse. As a result, the dermatitis symptom scores remained at significantly lower levels in the group that received a fermentation product of a propionic acid bacterium compared with the group that received distilled water (Fig. 1). On the other hand, the dermatitis symptom scores also remained at lower levels in the group that received a medium, but at higher levels compared with the group that received a fermentation product of a propionic acid bacterium.

For the total IgE in the serum, on day 14, the group that received a fermentation product of a propionic acid showed a significantly lower IgE concentration compared with the group that received distilled water (Fig. 2). On the other hand, the group that received a medium showed a lower concentration compared with the group that received distilled water, but no significant difference was observed.

For the SAA, on day 14, the group that received a fermentation product of a propionic acid bacterium (from Production Example 1 above) showed a significantly lower SAA concentration compared with the group that received distilled water (Fig. 3). On the other hand, the group that received a medium showed a lower concentration than the group that received distilled water, but no significant difference was observed. In contrast, on day 21, the group that received a fermentation product of a propionic acid bacterium showed a lower concentration than the group that received distilled water, but no significant difference was observed. On the other hand, the group that received a medium showed a significantly lower concentration than the group that received distilled water. On day 28, both the group that received a fermentation product of a propionic acid bacterium and the group that received a medium showed significantly lower concentrations than the group that received distilled water.

From the foregoing, the fermentation product of a propionic acid bacterium to be used according to the present invention was found to have high effects on the suppression of the development of atopic dermatitis.

### Test Example 2: Suppression test of passive cutaneous anaphylaxis reaction (PCA reaction) (animal experimental model of acute allergic reaction)

### (1) Outline

Male 7-week-old SD rats were preliminarily fed and then divided into the following four groups such that these groups had the same average body weight (n=7 or 8). The four groups include Group 1: a group that received distilled water; Group 2: a group that received a fermentation product of a propionic acid bacterium; Group 3: a group that received a medium; and Group 4: a group that received ketotifen fumarate (reagent for suppressing PCA reaction, manufactured by Wako Pure Chemical Industries, Ltd.). Hairs in the dorsal region of each rat were shaved with chippers, and then the region was provided with a PCA reaction induction site and a PCA reaction non-induction site. A solution of ananti-DNP-IgE antibody (25 ng/site, manufactured by ICN Pharmaceuticals, Inc.) and physiological saline were intradermally administered to the PCA reaction induction site and the PCA reaction non-induction site, respectively.

Various samples to be used in each group were the same as those in Test Example 1.

24 hours after the administration of the IgE antibody or physiological saline, each group was intravenously administered with DNP-bound BSA (DNP-BSA: 2,4-dinitrophenylated Bovine Serum Albumin) (1 mg/rat, manufactured by LSL Co., Ltd.) as an antigen together with Evans Blue (5 mg/rat, manufactured by Wako Pure Chemical Industries, Ltd.) to induce the PCA reaction. Each of the fermentation product of a propionic acid bacterium, medium, and distilled water was orally administered at 10 mL/kg (1.7 g/kg in terms of solid content) 1, 3, and 5 hours before the PCA reaction induction, and ketotifen fumarate was orally administered at 10 mg/kg 1 hour before the PCA reaction induction (3 and 5 hours before, distilled water was administered). The dorsal skin was obtained from the rat 30 minutes after the PCA reaction induction and treated with 1 N potassium hydroxide. After that, Evans Blue was extracted with a mixed solution of acetone and 0.6 N phosphoric acid, and the amount of Evans Blue leaked to the skin was calculated from an absorbance at 620 nm.

### (2) Results

Fig. 4 shows amounts of Evans Blue leakage. The amount of Evans Blue leakage was significantly lowered on the PCA reaction induction site of the group that received a fermentation product of a propionic acid bacterium [FERMENTATION PRODUCT OF PROPIONIC ACID BACTERIUM] compared with on the PCA reaction induction site of the group that received distilled water [DISTILLED WATER] (Fig. 4). On the other hand, the amount of Evans Blue leakage was also lowered on the PCA reaction induction site of the group that received a medium [MEDIUM], but no significant difference was observed.

From the foregoing, the fermentation product of a propionic acid bacterium to be used according to the present invention was found to have high effects on the suppression of the PCA reaction.

### Production Example 2: Production method for whey fermentation product of propionic acid bacterium

### 1. Material

### <Strain>

### Propionibacterium freudenreichii ET-3 strain (FERM BP-8115 strain)

### <Preculture medium and main culture medium>

A preculture medium and main culture medium were prepared in the same manner as the preparation method for a <preculture medium> in Production Example 1.

### 2. Production method

### <Preculture>

To 100 mL of the preculture medium, 1 mL of frozen ET-3 strain (stored at -80°C) was inoculated. A static culture was performed at 37°C for 48 hours under an anaerobic condition, and the culture solution was used as a starter in a main culture.

### <Main culture>

The main culture medium was inoculated with the preculture solution in an amount of 2% to start the main culture. The culture was continued at a culture temperature of 33 to 37°C for 66 to 96 hours under a nitrogen atmosphere to obtain a culture solution containing a propionic acid bacterium (a whey fermentation product of a propionic acid bacterium). In the preparation of Test meals in Test Example 3, the culture solution itself was used as a whey fermentation product of a propionic acid bacterium.

### Test Example 3: Clinical trial for human subjects with dry skin

### (1) Outline of test

### <Test subject>

A double-blind placebo-controlled trial was conducted with women of age 20 to 39 with dry skin. Prior to the trial, on the basis of cutaneous findings (dryness, a scale, a papule, a comedone, and a pustule) observed by a doctor, the subjects were divided into three groups so as not to generate bias. The trial was conducted under ethical niceties while complying with Declaration of Helsinki (revised at the Edinburgh meeting, 2000).

### <Test meal>

The following three kinds (Test meals A to C) were provided. The whey fermentation product of a propionic acid bacterium obtained in Production Example 2 above was used.

### Test meal A: an acidic beverage (placebo) [n=30]

Test meal A was obtained by adding various ingredients (pectin, lactic acid, yogurt flavor, vitamin C, and aspartame) to water.

Test meal A was free of milk ingredients and lactic acid bacteria.

### Test meal B: an acidic beverage containing a whey fermentation product of a propionic acid bacterium (Active 1) [n=28]

Test meal B was obtained by adding the whey fermentation product of a propionic acid bacterium to the above acidic beverage in an amount of 1 v/v%.

Test meal B was free of milk ingredients and lactic acid bacteria not derived from the whey fermentation product of a propionic acid bacterium.

### Test meal C: a yogurt beverage containing a whey fermentation product of a propionic acid bacterium (Active 2) [n=28]

Test meal C was obtained by incorporating a commercially-available yogurt in an amount of 60 v/v%, and adding water, various ingredients having the same concentration as those added to the above acidic beverage (placebo), sucrose, and the whey fermentation product of a propionic acid in an amount of 1 v/v%.

The DHNA content contained in 120 ml each of Test meal B and Test meal C is 13.2 µg.

### <Ingestion period and ingestion method of Test meal>

During the Test meal ingestion period from the ingestion starting day to four weeks later, 120 mL/day of the Test meal are orally ingested. During the Test meal ingestion period, the ingestion of a food containing lactic acid bacteria other than the Test meal (a yogurt, fermented milk, and the like) and a food containing oligosaccharide, the start of new supplement ingestion, the start of new treatment drug ingestion and external drug use for the purpose of beauty, and the spa treatment for the purpose of beauty (for example, chemical peeling) were abandoned.

### (3) Inspection items

Before the Test meal ingestion on the ingestion starting day of the Test meal, and 4 weeks after the start of the Test meal ingestion, the following inspection was performed.

Stratum corneum water content: a moisture meter, Corneometer CM825 (manufactured by Courage + Khazaka electronic GmbH (Germany)) was used to measure twice the stratum corneum water content of the face skin of the subject as a capacitance value, and their average value was calculated.

Skin texture: a skin image analyzer, Robo Skin Analyzer RSA-100 (manufactured by Inforward, inc) was used to score the face texture of the subject. In the present invention, the score value is referred to as texture score, and the higher texture score shows a condition where the skin texture is refined.

In addition, for the measurement value before and after the Test meal ingestion, a significant test was performed by using a comparison test in a group (paired t-test), and for the change amount before and after the Test meal ingestion, a significant test was performed by using a multiple comparison test among three groups (Tukey-Kramer's method).

### (4) Results

Figs. 5 and 6 show the results . For both the measurement values of the stratum corneum water content and the texture score, no significant difference was observed before and after the ingestion of Test meal A: an acidic beverage (placebo) . In contrast, after the ingestion of Test meal B: an acidic beverage containing a whey fermentation product of a propionic acid bacterium (Active 1) or Test meal C: a yogurt beverage containing a whey fermentation product of a propionic acid bacterium (Active 2), the increase in the measurement values of the stratum corneum water content and the texture score was significantly observed. Further, the change amounts of the measurement values of both the stratum corneum water content and the texture score before and after the ingestion of Test meals were significantly higher in a group that ingested Test meal B and a group that ingested Test meal C compared with a group that ingested Test meal A.

From the above results, the fermentation product of a propionic acid bacterium to be used according to the present invention was found to have an effect of improving dry skin conditions. Further, even when the fermentation product of a propionic acid bacterium to be used according to the present invention was used in combination with the yogurt, the same effect was obtained.

## Claims

1. A fermentation product of a propionic acid bacterium for use in improving dry skin conditions or skin roughness by oral administration or oral ingestion, wherein the fermentation product comprises a culture solution of a propionic acid bacterium.

2. The fermentation product of a propionic acid bacterium for the use according to claim 1, wherein the fermentation product comprises a culture solution of a propionic acid bacterium cultured by use of a medium containing a proteolytic enzyme-treated dairy product.

3. The fermentation product of a propionic acid bacterium for the use according to claim 2, wherein the dairy product is one or more kinds selected from skim milk, whey powder, and a milk protein.

4. The fermentation product of a propionic acid bacterium for the use according to any one of claims 1 to 3, wherein the propionic acid bacterium is a propionic acid bacterium for cheese.

5. The fermentation product of a propionic acid bacterium for the use according to claim 4, wherein the propionic acid bacterium for cheese is *Propionibacterium freudenreichii.*

6. The fermentation product of a propionic acid bacterium for the use according to any one of claims 1 to 5, wherein the fermentation product comprises a propionic acid bacterium and the culture solution of the propionic acid bacterium.

## Patentansprüche

1. Ein Fermentationsprodukt eines Propionsäurebakteriums zur Verwendung zur Verbesserung von trockenen Hautzuständen oder von Hautrauheit durch orale Verabreichung oder orale Einnahme , wobei das Fermentationsprodukt eine Kulturlösung eines Propionsäurebakteriums umfasst.

2. Das Fermentationsprodukt eines Propionsäurebakteriums zur Verwendung gemäß Anspruch 1, wobei das Fermentationsprodukt eine Kulturlösung eines Propionsäurebakteriums umfasst, das unter Verwendung eines Mediums kultiviert wurde, das ein mit proteolytischem Enzym behandeltes Milchprodukt enthält.

3. Das Fermentationsprodukt eines Propionsäurebakteriums zur Verwendung gemäß Anspruch 2, wobei das Milchprodukt eine oder mehrere Arten ist, ausgewählt aus Magermilch, Molkepulver und einem Milchprotein.

4. Das Fermentationsprodukt eines Propionsäurebakteriums zur Verwendung gemäß einem der Ansprüche 1 bis 3, wobei das Propionsäurebakterium ein Propionsäurebakterium für Käse ist.

5. Das Fermentationsprodukt eines Propionsäurebakteriums zur Verwendung gemäß Anspruch 4, wobei das Propionsäurebakterium für Käse *Propionibacterium freudenreichii* ist.

6. Das Fermentationsprodukt eines Propionsäurebakteriums zur Verwendung gemäß einem der Ansprüche 1 bis 5, wobei das Fermentationsprodukt ein Propionsäurebakterium und die Kulturlösung des Propionsäurebakteriums umfasst.

## Revendications

1. Produit de fermentation d'une bactérie propionique pour une utilisation dans l'amélioration des conditions de sécheresse cutanée ou de rugosité cutanée par administration orale ou ingestion orale, lequel produit de fermentation comprend une solution de culture d'une bactérie propionique.

2. Produit de fermentation d'une bactérie propionique pour une utilisation selon la revendication 1, lequel produit de fermentation comprend une solution de culture d'une bactérie propionique cultivée par utilisation d'un milieu contenant un produit laitier traité par une enzyme protéolytique.

3. Produit de fermentation d'une bactérie propionique pour une utilisation selon la revendication 2, dans lequel le produit laitier est d'un ou plusieurs types choisis parmi le lait écrémé, la poudre de lactosérum, et les protéines de lait.

4. Produit de fermentation d'une bactérie propionique pour une utilisation selon l'une quelconque des revendications 1 à 3, dans lequel la bactérie propionique est une bactérie propionique pour le fromage.

5. Produit de fermentation d'une bactérie propionique pour une utilisation selon la revendication 4, dans lequel la bactérie propionique pour le fromage est *Propionibacterium freudenreichii.*

6. Produit de fermentation d'une bactérie propionique pour une utilisation selon l'une quelconque des revendication 1 à 5, lequel produit de fermentation comprend une bactérie propionique et la solution de culture de la bactérie propionique.
